# DEMANDE DE BREVET EUROPEEN

(11) **EP 1 994 921 A1**
(43) Date de publication de la demande: **26.11.2008**
(21) Numéro de dépôt: 08156547.5
(22) Date de dépôt: 20.05.2008
(51) Int. Cl.: A61K 8/37, A61K 8/41, A61K 8/49, A61Q 13/00

(54) **Composition parfumante comprenant l'association d'un filtre A hydroxyaminobenzophenone, d'un filtre B cinnamate et d'un composé C pipéridinol, benzotriazole ou dibenzoylméthane**

(30) Priorité: 21.05.2007 FR 0755153; 21.05.2007 FR 0755154; 21.05.2007 FR 0755155; 21.05.2007 FR 0755152
(71) Demandeur: L'Oreal, 75008 Paris (FR)
(72) Inventeur: Bara, Isabelle, 94210, La Varenne St Hilaire (FR)
(74) Mandataire: Miszputen, Laurent

(57) **Abrégé**

L'invention se rapporte à une L'invention se rapporte à une composition parfumante comprenant dans un milieu cosmétiquement acceptable :
a) au moins 2% en poids d'une substance parfumante par rapport au poids total de la composition ;
b) au moins un filtre A hydroxyaminobenzophenone de formule (1) particulière que l'on définira plus loin en détail ;
c) au moins un filtre B cinnamate ;
d) au moins un composé C choisi parmi :
(i) un composé pipéridinol de formule (II) particulière que l'on définira plus loin en détail ;
(ii) au moins un composé benzotriazole de formule (III) particulière que l'on définira plus loin en détail
(iii) un filtre UV dibenzoylméthane éventuellement en association avec au moins un filtre D salicylate.

L'invention se rapporte également à l'utilisation de cette association b), c) et d) dans une composition parfumante comprenant dans un milieu cosmétiquement acceptable au moins 2% en poids d'une substance parfumante par rapport au poids total de la composition ; comme agent stabilisant des propriétés organoleptiques de la composition vis-à-vis des agressions extérieures comme la lumière ou les différences de température en particulier de la couleur et/ou de l'odeur de ladite composition

L'invention concerne également à un procédé cosmétique de parfumage des matières kératiniques humaines et, comprenant l'application sur les matières kératiniques de ladite composition parfumante.

## Description

L'invention se rapporte à une composition parfumante comprenant dans un milieu cosmétiquement acceptable :
a) au moins 2% en poids d'une substance parfumante par rapport au poids total de la composition ;
b) au moins un filtre A hydroxyaminobenzophenone de formule (I) particulière que l'on définira plus loin en détail ;
c) au moins un filtre B cinnamate ;
d) au moins un composé C choisi parmi:
   (i) un composé pipéridinol de formule (II) particulière que l'on définira plus loin en détail ;
   (ii) au moins un composé benzotriazole de formule (III) particulière que l'on définira plus loin en détail
   (iii) un filtre UV du type dibenzoylméthane.

On sait qu'un parfum est l'association de différentes substances odorantes qui s'évaporent à des périodes différentes. Chaque parfum présente ce que l'on appelle une « note de tête » qui est l'odeur diffusant en premier lors de l'application du parfum ou lors de l'ouverture du récipient le contenant, une « note de coeur ou corps » qui correspond au parfum complet (émission pendant quelques heures après la « note de tête ») et une « note de fond » qui est l'odeur la plus persistante (émission pendant plusieurs heures après la « note de coeur »). La persistance de la note de fond correspond à la rémanence du parfum.

L'être humain a de tout temps cherché à se parfumer et à parfumer les objets qui l'entourent ou les lieux dans lesquels il se trouve, et ceci, aussi bien pour masquer des odeurs fortes et/ou désagréables que pour donner une bonne odeur.

Il est courant d'incorporer du parfum dans un certain nombre de produits ou compositions, en particulier cosmétiques et dermatologiques telles que des eaux fraîches, eaux de toilette, des eaux de parfum, des lotions après rasage, des eaux de soin.

Un parfum ou une composition parfumante doit avoir une odeur agréable et une couleur stables selon un certain temps correspondant au temps d'utilisation de la consommatrice. Ils doivent résister à différentes agressions telles que la lumière et des différences de température.

On ajoute généralement un système filtrant et/ou un système antioxydant. Cependant, certains systèmes filtrants sont inefficaces et/ou ont tendance à dénaturer la couleur et/ou le parfum recherchés.

La plupart des produits parfumants comportent des stabilisateurs tels que des filtres UVA ou filtre UVB et des anti-oxydants.

Les stabilisateurs ont pour rôle de maintenir les propriétés organoleptiques des produits stables à différentes agressions telles que les UV et les chocs thermiques. Des agents stabilisants sont connus de l'art antérieur pour améliorer la résistance de ces produits vis-à-vis de ces agressions.

On connaît dans la demande de brevet WO2005/123013 l'association du diethylamino hydroxybenzoylhexylbenzoate et de l'éthyl hexyl methoxycinnamate, vendu sous la dénomination Uvinul A + B, comme stabilisant la couleur dans les compositions parfumantes.

On connaît également dans la demande de brevet WO2000/25730 utilisant le Tinogard AS ou " Bumetrizole " pour éviter les dégradations photolytiques des produits cosmétiques, dont les parfums.

On connaît également dans la demande de brevet WO2003/103622 l'utilisation du Tinogard Q ou "tris (Tetramethylhydroxypiperidinol) pour stabiliser des parfums, dont les eaux de toilette.

Le brevet WO 2005/042828 cite l'association Tinogard Q à des filtres particuliers dans le but de stabiliser des parfums et éviter la dégradation des eaux de toilette à la lumière.

Malheureusement les filtres UV ou les stabilisants préconisés par les demandes de brevet de l'art antérieur ne sont pas pleinement satisfaisants, en particulier pour les parfums fragiles riches en substances naturelles. On observe le plus souvent des modifications des propriétés organoleptiques comme en particulier celles de l'odeur et de la couleur au cours du temps (jugées par analyse sensorielle tels que le "sniff test" pour l'odeur, et un test de visualisation à l'oeil nu ou par un spectromètre pour quantifier les évolutions de couleur selon différents paramètres de l'évolution de la couleur, selon l'exposition à différents types de clarté). En particulier, dans les modifications visuelles, apparaissent facilement des jaunissements, des rosissements liés à la dégradation des corps chimiques. On peut voir également des modifications de clarté des liquides, lorsque ceux-ci sont à l'origine transparents, telles que des floculations et des précipités de matières ou des troubles altérant l'aspect des produits et pouvant nuire à l'efficacité des pompes, lorsque le produit est conditionné en spray.

Aussi, il subsiste donc le besoin de rechercher de nouveaux produits parfumants ne présentant pas les inconvénients des produits de l'art antérieur, et notamment le besoin de produits parfumants dont les propriétés organoleptiques comme l'odeur et la couleur restent stables dans le temps et sous les effets de la lumière ou des différences de température.

La demanderesse a découvert de manière surprenante que cet objectif pouvait être atteint en utilisant une composition parfumante comprenant dans un milieu cosmétiquement acceptable :
a) au moins 2% en poids d'une substance parfumante par rapport au poids total de la composition ;
b) au moins un filtre A hydroxyaminobenzophenone de formule (I) particulière que l'on définira plus loin en détail ;
c) au moins un filtre B cinnamate ;
d) au moins un composé C choisi parmi :
   (i) un composé pipéridinol de formule (II) particulière que l'on définira plus loin en détail ;
   (ii) au moins un composé benzotriazole de formule (III) particulière que l'on définira plus loin en détail
   (iii) un filtre UV du type dibenzoylméthane ;

Cette découverte est à la base de l'invention.

L'invention se rapporte également à l'utilisation cosmétique de l'association d'au moins un filtre A hydroxyaminobenzophenone de formule (I) particulière que l'on définira plus loin en détail , d'au moins un filtre B cinnamate et d'au moins un composé C choisi parmi:
(i) un composé pipéridinol de formule (II) particulière que l'on définira plus loin en détail ;
(ii) au moins un composé benzotriazole de formule (III) particulière que l'on définira plus loin en détail
(iii) un filtre UV du type dibenzoylméthane ;
dans une composition parfumée comprenant dans un milieu cosmétiquement acceptable au moins 2% en poids d'une substance parfumante ; comme agent stabilisant des propriétés organoleptiques en particulier de la couleur et/ou de l'odeur de ladite composition vis-à-vis des agressions extérieures comme la lumière ou les différences de température.

L'invention a encore pour objet un procédé cosmétique de parfumage des matières kératiniques humaines et notamment de la peau, des lèvres, des cheveux, du cuir chevelu, les lèvres, les ongles, comprenant l'application sur les matières kératiniques de la composition telle que définie ci-dessus.

Par composition parfumante, on entend toute composition laissant après application sur les matières kétatiniques un parfum.

Par « substance parfumante», on entend tout parfum ou arôme susceptible de parfumer la peau et les matières kératiniques humaines en général comprenant la peau, les cheveux, le cuir chevelu, les lèvres ; les ongles.

La quantité de substance(s) parfumante(s) sera plus préférentiellement de 3 à 50 % en poids, mieux de 5 à 30%, encore mieux 10 à 20% en poids par rapport au poids total de la composition.

On entend par milieu cosmétiquement acceptable dans la composition de l'invention, un milieu non toxique et susceptible d'être appliqué sur les matières kératiniques humaines comprenant la peau, le visage, les lèvres, les ongles, les cheveux, le cuir chevelu.

Comme substance parfumante, on peut utiliser dans la composition de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance parfumante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcool aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On utilise de préférence comme substance parfumante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Selon un mode préféré de réalisation de l'invention, on utilise un mélange de différentes substances parfumante qui engendrent en commun une note plaisante pour l'utilisateur. Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges

Les composés A hydroxyaminobenzophenones conformes à l'invention répondent à la formule générale (I) suivante : 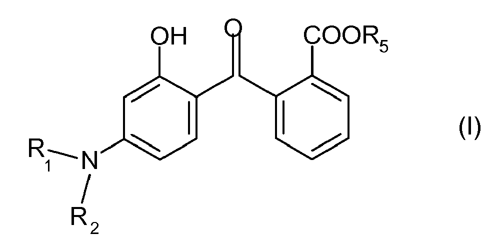 dans laquelle :
R₁ et R₂, identiques ou différents, désignent un atome d'hydrogène, un radical alkyle en C₁-C₂₀ ; un cycloalkyle en C₃-C₁₀ ou un cycloalcényle en C₃-C₁₀ ou forment avec l'atome d'azote avec lequel ils sont liés un cycle à 5 ou 6 chaînons ;
R₃ désigne un radical alkyle en C₁-C₂₀.

Comme radicaux alkyle en C₁-C₂₀, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme radicaux cycloalkyles en C₃-C₁₀, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclypropyle, 1-méthyl-2-éthylcyclopropyle, cyclooctyle, cyclononyle ou cyclodécyle.

Comme radicaux cycloalcènyles en C₃-C₁₀ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Comme exemples de cycle à 5 ou 6 chaînons formé par les radicaux R₁ et R₂ avec l'atome d'azote, on peut citer en particulier pyrrolidine ou pipéridine.

Un composé A de formule (I) tout particulièrement préféré est le 2-(4-diéthylamino-2-hydroxybenzoyl)-benzoate de n-hexyle de formule (A) : 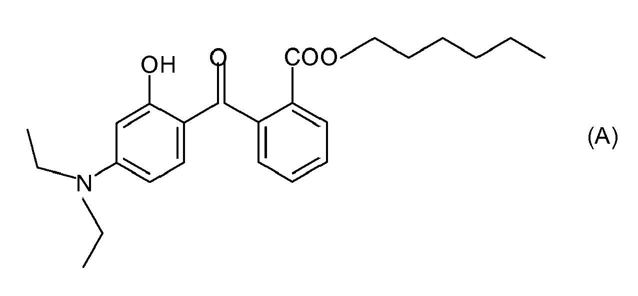 tel que le produit vendu sous le nom commercial « UVINUL A+ » par la société BASF.

Les composés A de formule (I) tels que définis ci-dessus sont connus en eux-mêmes et leurs structures et leurs synthèses sont décrites dans les demandes de brevet EP-A-1046391, EP1133980 et DE100 12 408.

De préférence, le composé A hydroxyaminobenzophenone de formule (I) est présent dans les compositions selon l'invention à une teneur allant de 0,01% à 1,5% en poids, et plus particulièrement de 0,1 à 0,8% en poids par rapport au poids total de la composition.

Parmi les filtres B cinnamate conformes à l'invention, on peut citer notamment de manière non limitative :
Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
Diisopropyl Methylcinnamate,
Glyceryl Ethylhexanoate Dimethoxycinnamate

Parmi les filtres B cinnamates mentionnés ci-dessus, on utilisera tout particulièrement Ethylhexyl Methoxycinnamate proposé à la vente sous la dénomination commerciale de "PARSOL MCX" par la Société DSM ; ce filtre répond à la formule (B) suivante :

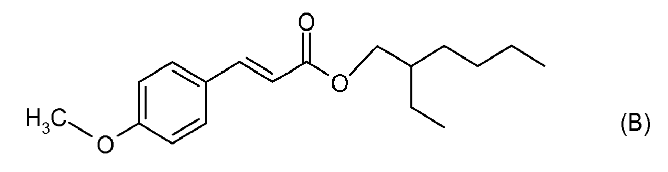

De préférence, le composé B cinnamate est présent dans les compositions selon l'invention à une teneur allant de 0,01 % à 1,5% en poids, et plus particulièrement de 0,1 à 0,8% en poids par rapport au poids total de la composition.

Selon une forme particulière de l'invention, on utilisera l'association 2-(4-diéthylamino-2-hydroxybenzoyl)-benzoate de n-hexyle / Ethylhexyl Methoxycinnamate dans un rapport en poids 35/65% en poids tel que le produit vendu sous le nom commercial « UVINUL A +B »par BASF.

Cette association particulière sera présente dans les compositions selon l'invention à une teneur allant de 0,01% à 2% en poids, et plus particulièrement de 0,1 à 1% en poids et encore plus particulièrement de 0,3 à 1% en poids par rapport au poids total de la composition par rapport au poids total de la composition.

Le composé C conforme à l'invention est choisi parmi
(i) un composé pipéridinol de formule (II) particulière que l'on définira plus loin en détail ;
(ii) au moins un composé benzotriazole de formule (III) particulière que l'on définira plus loin en détail
(iii) un filtre UV dibenzoylméthane ;

Les composés pipéridinols conformes à l'invention répondent à la formule suivante (II) : 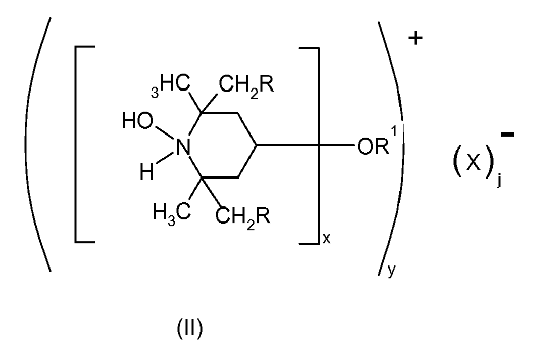 dans laquelle
R désigne hydrogène ou méthyle
x vaut 1 ou 2
1) lorsque x est égal à 1 :
   R¹ désigne un hydrogène ; un radical alkyle en C₁-C₁₈ un radical alcényle en C₂-C₁₈ un radical propargyle ; un groupe glycidyle ; un radical alkyle en C₂-C₅₀ interrompu par 1 à 20 atomes d'oxygène, ledit alkyle étant substitué par 1 à 10 groupes hydroxyles ou bien à la fois interrompu par lesdits atomes d'oxygène et substitué par lesdits groupes hydroxyles ; un radical alkyle en C₁-C₄ substitué par un groupe carboxy ou un groupe -COOZ où Z est hydrogène, un alkyle en C₁-C₄, phényle, un alkyle en C₁-C₄ substitué par un groupe (COO ⁻) ₚ M ^{p+} où p est un entier de 1 à 3 et M un ion métallique des groupes 1, 2 et 3 du tableau périodique ou Zn , Cu, Ni ou Co ou bien M est un groupe N^{p+} (R²)₄ où R² est un alkyle en C₁-C₈ ou un benzyle ;
2) lorsque x vaut 2 ,
   R' est un radical alkylène en C₁-C₁₂; un radical alcénylène en C₄-C₁₂; un groupe xylylène ; un radical alkylène en C₁-C₅₀ interrompu par 1 à 20 atomes d'oxygène ledit alkyle étant substitué par 1 à 10 groupes hydroxyles ou bien à la fois interrompu par lesdits atomes d'oxygène et substitué par lesdits groupes hydroxyles ; X désigne un anion organique ou minéral ;
   la charge totale en cations y étant égal à la charge totale en anions j.

Parmi les anions X, on peut citer phosphate, phosphonate, carbonate, bicarbonate, nitrate, chlorure, bromure, bisulfite, sulfite, bisulfate, sulfate, borate, formate,acétate, benzoate, citrate, oxalate, tartrate, acrylate, polyacrylate, fumarate, maléate, itaconate, glycolate, gluconate, malate, mandélate, tiglate, ascorbate, polyméthacrylate, un carboxylate de l'acide nitrilotriacétique, l'acide hydroxyéthylènediaminetriacétique, l'acide éthylènediaminetétraacétique, l'acide diéthylènediaminepentaacétique, le diéthylènetriaminepentaméthylènephosphonate, un alkylsulfonate ou un arylsulfonate.

Les composés pipéridinols de formule (II) sont connus en eux-mêmes et décrits dans la demande WO03/103622.

On utilisera en particulier les composés de formule (II) pour lesquels R et R¹ désignent l'hydrogène, x = 1 et X désigne l'anion citrate et encore plus particulièrement le composé Tris-(tétraméthylhydroxypipéridinol) citrate de structure : 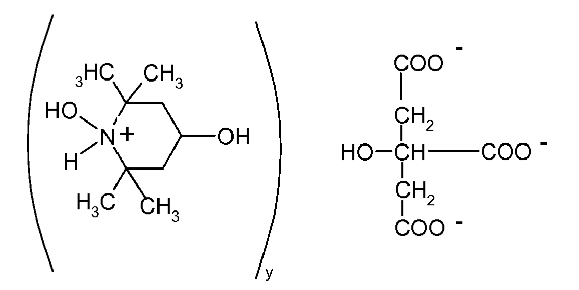 avec y = 3 tel que le produit commercial vendu le nom TINOGUARD Q ou TINOGUARD S-FX par la société CIBA- GEIGY.

Les composés benzotriazole conformes à l'invention répondent à la formule suivante : 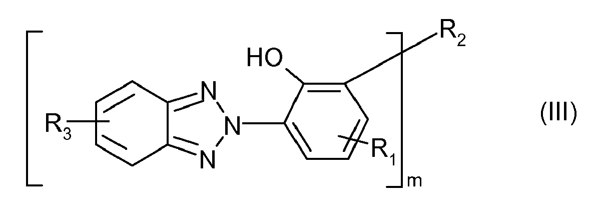 dans laquelle
R₁ désigne un radical alkyle en C₁-C₁₂, un radical alcoxy en C₁-C₅, un alcoxycarbonyle en C₁-C₅, un cycloalkyle en C₅-C₇, un aryle en C₆-C₁₀, un radical aralkyle, un groupe SO₃M, un radical de formule (IIa) 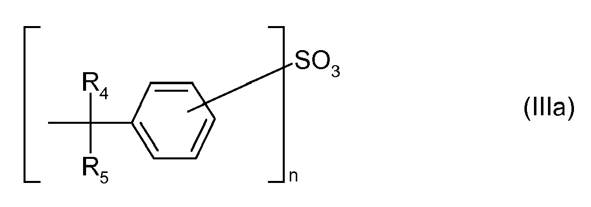 R₄ et R₅, identiques ou différents désignent un alkyle en C₁-C₅ ou un hydrogène ;
R₃ désigne hydrogène, un radical alkyle en C₁-C₁₂, un radical alcoxy en C₁-C₅, un halogène de préférence Cl ou un groupe hydroxy ;
M désigne un cation métallique alcalin, alcalino-terreux ou ammonium de préférence Na+ m désigne 1 ou 2
n vaut 0 ou 1
si m = 1 alors R₂ est hydrogène, un groupe alkyle en C₁-C₁₂ substitué ou non par un phényle ; un aryle en C₆-C₁₀ ;
si m = 2 alors R₂ est une liaison directe, un radical -(CH₂) p- avec p étant un entier de 1 à 3.

Les radicaux alkyle sont linéaires ou ramifiés et sont choisis par exemple parmi méthyle, éthyle, n-propyle, isopropyle, n-butyle, sec-butyle, tert-butyle, amyle, isoamyle, heptyle, octyle, isooctyle, nonyle, décyle, dodécyle.

Comme radicaux alcoxy en C₁-C₅, on peut citer : méthoxy, éthoxy, n-propoxy, n-butoxy, n-pentoxy, 1-méthylpropoxy, 3-méthylbutoxy, 2,2-diméthylpropoxy.

Les cycloalkyle en C₅-C₇ sont par exemple cyclopentyle, cycloheptyle, ou de préférence cyclohexyle.

Les alkylphenyles sont par exemple phénylpropyle, phényléthyle et de préférence benzyle.

Les composés benzotriazoles de formule (II) sont connus en eux-mêmes et leurs structures sont décrites dans la demande WO 00/25730

Parmi les composés de formule (III), on peut citer notamment les composés suivants :
(i) Sodium Benzotriazolyl Btylphenol Sulfonate de formule (1) 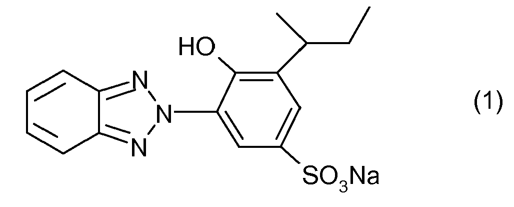 comme le produit vendu sous le nom « TINOGUARD HS » par la société CIBA-GEIGY.
(ii) Benzotriazolyl dodécyl p-Cresol de formule (2) : 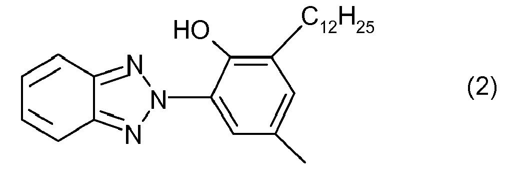 comme le produit vendu sous le nom « TINOGUARD TL » par la société CIBA-GEIGY.
(iii) Benzotriazolyl Butylphenol Sulfonate de formule (3) 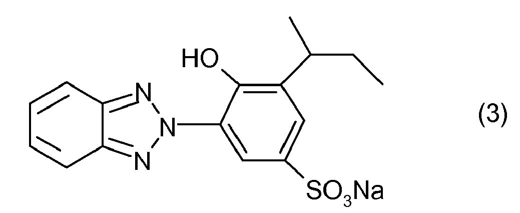 comme le produit vendu sous le nom commercial « CIBAFAST H LIQUID » par la société CIBA-GEIGY.
(iv) Bumetrizole de formule (4) : 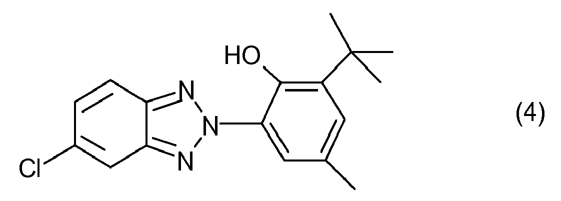 comme le produit vendu sous le nom « TINOGUARD AS » par la société CIBA-GEIGY.

On utilisera plus particulièrement le composé Bumetrizole de formule (4).

Le ou les composés C du type pipéridinol ainsi que ceux du type benzotriazole conformes à l'invention sont de préférence présents dans les compositions selon l'invention à une teneur allant de 0,01 à 0,1 %de la composition finale, plus préférentiellement de 0,01 à 0,05% en poids et encore plus particulièrement de 0,01 à 0,03 % en poids par rapport au poids total de la composition.

Parmi les filtres dibenzoylméthane conformes à l'invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4,4'-diméthoxydibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert-butyl-4'-méthxydibenzoylméthane,
- le 2,4-diméthyl-4'-méthoxydibenzoylméthane,
- le 2,6-diméthyl-4-tert-butyl-4'-méthoxydibenzoylméthane.

Parmi les dibenzoylméthanes mentionnés ci-dessus, on utilisera tout particulièrement le 4-(ter.-butyl) 4'-méthoxy dibenzoylméthane ou Butyl Methoxy Dibenzoylmethane, proposé à la vente sous la dénomination commerciale de "PARSOL 1789" par la Société DSM ; ce filtre répond à la formule (IV) suivante :

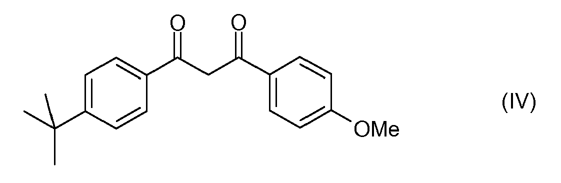

Le ou les filtres dibenzoylméthane conformes à l'invention sont de préférence présents dans les compositions selon l'invention à une teneur allant de 0,01% à 1,5% en poids, de préférence de 0,1 à 0,5% en poids et plus particulièrement de 0,1 à 0,8% en poids par rapport au poids total de la composition.

Selon une forme particulière de l'invention, la composition parfumante contient dans un milieu cosmétiquement acceptable :
a) au moins 2% en poids d'une substance parfumante par rapport au poids total de la composition ;
b) au moins un filtre A hydroxyaminobenzophenone de formule (I) tel que défini précédemment ;
c) au moins un filtre B cinnamate tel que défini précédemment ;
d) au moins un filtre C dibenzoylméthane tel que défini précédemment et
e) au moins un filtre D du type salicylate.

Parmi les filtres D du type salicylate conformes à l'invention, on peut notamment citer, par exemple :
Homosalate vendu sous le nom « Eusolex HMS » par Rona/EM Industries,
Ethylhexyl Salicylate vendu sous le nom « NEO HELIOPAN OS » par SYMRISE,
Dipropyleneglycol Salicylate vendu sous le nom « DIPSAL » par SCHER,
TEA Salicylate, vendu sous le nom « NEO HELIOPAN TS » par SYMRISE,

Parmi les salicylates D mentionnés ci-dessus, on utilisera tout particulièrement l' Ethylhexyl Salicylate vendu sous le nom « NEO HELIOPAN OS » par SYMRISE de formule (V) suivante :

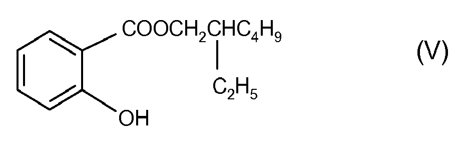

Le ou les filtres D du type salicylate conformes à l'invention sont de préférence présents dans les compositions selon l'invention à une teneur allant de 0,01% à 1,5% en poids, de préférence de 0,1 à 0,5% en poids et plus particulièrement de 0,1 à 0,8% en poids par rapport au poids total de la composition.

Selon une forme particulière de l'invention, le milieu cosmétiquement acceptable conforme à la présente invention contient au moins un alcool volatil et/ou une huile de silicone volatile et éventuellement de l'eau. De façon préférentielle ce milieu de la composition contient de l'eau dans une quantité allant de préférence de 0,01 % à 50 % et plus préférentiellement, de 0,5 % à 25 % en poids par rapport au poids total de la composition.

Par « alcool volatil », tout composé comprenant au moins une fonction alcool ayant une pression de vapeur à 20 °C supérieure à 17,5 mm de mercure.

Par « huile », on entend, au sens de l'invention, un corps gras, non soluble dans l'eau, et liquide à température ambiante et pression atmosphérique.

Par « huile de silicone volatile », on entend, au sens de l'invention, tout composé siliconé susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa (10⁻³ à 300 mm de Hg), en particulier allant de 1,3 Pa à 13 000 Pa (0,01 à 100 mm de Hg), et plus particulièrement allant de1,3 Pa à 1300 Pa (0,01 à 10 mm de Hg).

Les alcools volatils conformes à la présente invention sont choisis de préférence parmi monoalcools inférieurs en C₁-C₅ peuvent être choisis parmi le méthanol, l'éthanol, propanol, l'isopropanol, le n-butanol, l'isobutanol le t-butanol et plus particulièrement l'éthanol.

Le ou les alcools volatils sont présents de préférence dans des quantités allant de 40 à 80% et plus préférentiellement dans des quantités allant de 55 à 80% en poids par rapport au poids total de la composition.

Comme huiles siliconées volatiles, on peut citer par exemple les huiles de silicones linéaires ou cycliques volatiles, notamment celles ayant une viscosité ≤ 6 centistokes (6.10⁻⁶ m²/s), et ayant notamment de 2 à 10 atomes de silicium, ces silicones comportant éventuellement des groupes alkyles ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile ou les huiles siliconées volatiles sont présentes de préférence de 40 à 98,5% de préférence dans des concentrations allant de 10% à 80 % en poids par rapport au poids total de la composition.

La composition de l'invention peut comprendre, en outre, tout additif usuellement utilisé dans le domaine des parfums choisi notamment parmi les antioxydants, les actifs cosmétiques ou dermatologiques comme par exemple les émollients ou adoucissants comme les huiles d'amande douce, de noyau d'abricot, les agents hydratants comme la glycérine, les agents apaisants comme l'α-bisabolol, l'allantoïne, aloes vera ; les vitamines, les acides gras essentiels, les agents répulsifs contre les insectes, les propulseurs, les peptisants,, des matières colorantes des nacres, des paillettes et leurs mélanges. La composition de l'invention peut contenir également des adjuvants habituels dans le domaine cosmétique ou dermatologique tels que les gélifiants hydrophiles ou lipophiles, les conservateurs (par exemple phenoxyethanol et parabens), les solvants, les charges, les bactéricides. Les quantités de ces différents adjuvants sont celles classiquement utilisées dans le domaine considéré, et par exemple de 0,01 à 20 % du poids total de la composition.

Parmi les antioxydants, on peut citer par exemple le BHA (tert-butyl-4-hydroxyanisole), le BHT (2,6-di-tert-butyl-p-crésol), les tocophérols comme la vitamine E et ses dérivés tels que l'acétate de tocophéryle.

La composition selon l'invention peut notamment comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants solubles sont par exemple : le caramel, Yellow 5 , Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (CI 15850, CI 75470), Ext. Violet 2, Red 6-7, Ferric Ferrocyanide, Ultramarines, Acide Yellow 3 / Yellow 10, Acid Blue 3, Yellow 10.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

L'invention s'applique non seulement aux produits parfumants mais aussi aux produits de soin, de traitement du corps, de la peau, du visage, des lèvres, des ongles y compris des cheveux et du cuir chevelu contenant une substance odorante. La composition selon l'invention peut ainsi constituer une composition de parfumage, de soin, de traitement des matières kératiniques, et notamment se présenter sous forme d'eau fraîche, d'eau de toilette, d'eau de parfum, de lotion après-rasage, d'eau de soin, d'huile de soin siliconée ou hydrosiliconée, de produits d'hygiène comme les gels douvhes, les produits pour le bain, les shampooings, les scrubs. Elle peut également se présenter sous la forme d'une lotion biphasique ou tri-phasique parfumante (phase eau de toilette/phase huile hydrocarbonée et/ou huile siliconée et/ou huile fluorée).

L'invention a encore pour objet un procédé cosmétique de parfumage des matières kératiniques des êtres humains et notamment de la peau, des lèvres et des phanères, comprenant l'application sur les matières kératiniques de la composition telle que définie ci-dessus.

La composition selon l'invention peut être fabriquée par les procédés connus, généralement utilisés dans le domaine des compositions parfumées.

Les compositions selon l'invention peuvent être conditionnées sous forme de flacons. Elles peuvent également être appliquées sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane.

Les compositions parfumantes selon l'invention se présenter sous toutes les formes galéniques classiquement utilisées pour une application topique et notamment sous forme de solutions aqueuse ou hydroalcooliques, d'émulsions huile-dans-eau (H/E) ou eau-dans-huile (E/H) ou multiple (triple : E/H/E ou H/E/H), de gels aqueux, de produits anhydres deshydratés comme des poudres parfumantes libres ou compactes, ou de dispersions d'une phase huileuse dans une phase aqueuse à l'aide de vésicules lipidiques de type ionique (liposomes) et/ou non ionique. Ces compositions sont préparées selon les méthodes usuelles.

En outre, les compositions selon l'invention peuvent être plus ou moins fluides et avoir l'aspect d'une crème, d'une pommade, d'un lait, d'une lotion, d'un sérum, d'une pâte, d'une mousse. Elles peuvent aussi se présenter sous forme solide, et par exemple sous forme de stick.

Quand la composition selon l'invention comporte une phase huileuse, celle-ci contient de préférence au moins une huile, notamment une huile physiologiquement acceptable. Elle peut contenir en outre d'autres corps gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R₁OR₂ dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90R par la société Goldschmidt et le mélange de Polyglyceryl-4 isostearate/Cetyl dimethicone copolyol/Hexyl laurate vendu sous la dénomination Abil WE 09R par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; et leurs mélanges tels que le mélange de mono-stéarate de glycéryle et de stéarate de polyéthylène glycol (100 OE) commercialisé sous la dénomination SIMULSOL 165 par la société SEPPIC ; les esters d'acides gras et de sorbitan oxyalkylénés comprenant par exemple de 20 à 100 OE, et par exemple ceux commercialisés sous les dénominations commerciales Tween 20 ou Tween 60 par la société Ubiqema ; les éthers d'alcools gras oxyalkylénés (oxyéthylénés et/ou oxypropylénés) ; les esters de sucres comme le stéarate de sucrose ; et leurs mélanges comme par exemple le mélange de stéarate de glycéryle et de stéarate de PEG-100, commercialisé sous la dénomination Arlacel 165 par la société Uniqema.

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique.

On peut aussi préparer des émulsions sans tensioactifs émulsionnants ou en contenant moins de 0,5 % du poids total de la composition, en utilisant des composés appropriés, par exemple les polymères ayant des propriétés émulsionnantes tels que les polymères commercialisés sous les dénominations Carbopol 1342 et Pemulen par la société Noveon ; ou les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ou comme le polymère en émulsion commercialisé sous la dénomination Sepigel 305 par la société Seppic (nom INCI : Polyacrylamide / C13-C14 isoparaffine / laureth-7); les particules de polymères ioniques ou non ioniques, plus particulièrement des particules de polymère anionique comme notamment les polymères d'acide isophtalique ou d'acide sulfoisophtalique, et en particulier les copolymères de phtalate / sulfoisophtalate / glycol (par exemple diéthylèneglycol / Phtalate / isophtalate/1,4-cyclohexane-diméthanol (nom INCI : Diglycol/ CHDM/I sophtalates/ SIP Copolymer) vendus sous les dénominations Eastman AQ polymer (AQ35S, AQ38S, AQ55S, AQ48 Ultra) par la société Eastman Chemical.

On peut aussi préparer des émulsions sans émulsionnants, stabilisées par des particules siliconées ou des particules d'oxyde métallique tels que TiO₂ ou autres.

Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

L'invention va maintenant être décrite en référence aux exemples suivants donnés à titre illustratif et non limitatif. Dans ces exemples, sauf indication contraire, les quantités sont exprimées en pourcentages pondéraux. On a réalisé les formulations parfumées suivantes ; les quantités sont indiquées en pourcentages en poids :

### Exemples

### TESTS COMPARATIFS DE STABILITE DE L'ODEUR ET DE LA COULEUR

### Principe

Le principe de ces essais consiste à exposer les produits à tester à une source lumineuse irradiante dont la répartition spectrale est parfaitement définie et l'énergie émise parfaitement quantifiée.

Les sources lumineuses couramment utilisées sont des lampes au xénon émettant au travers :
- un filtre en quartz platiné qui détourne le rayonnement infra rouge et l'élimine vers le haut de l'appareil
- et un filtre en verre, qui en absorbant le rayonnement ultra violet court permet de simuler le rayonnement reçu derrière une vitrine d'exposition.

### Matériel

On utilise un appareil CPS Sun -test :
- L'éclairement fourni par une lampe au xénon entre 300 et 800nm est fixé à 765W/m² (valeur réglée par le constructeur)
- La filtration optique est assurée par d'un filtre quartz avec revêtement IR et un verre à vitres spécial)

On observe à l'oeil nu la couleur de chaque flacon avant et après exposition à la lumière au Sun Test après 16 heures à température ambiante.

On effectue également un test olfactif « sniff test » sur un panel de 5 personnes sur les échantillons à T₀ et après 2 mois à température ambiante et à 45°C.

### Exemples 1a, 1b et 1c

| **Composition** | **1a(*)** | **1b(*)** | **1c (invention)** |
|---|---|---|---|
| Concentré de parfum x | 15 % | 15 % | 15 % |
| Ethanol | 65% | 65% | 65% |
| Colorants | qs | qs | qs |
| Mélange de 2-(4-diéthylamino- | 1 | 0 % | 1 |
| 2-hydroxybenzoyl)-benzoate de | | | |
| n-hexyle et de Ethylhexyl | | | |
| Methoxycinnamate 35/65 | | | |
| (Uvinul A + B) | | | |
| Tris- | 0 | 0,03 | 0,03 |
| (tétraméthylhydroxypipéridinol) | | | |
| citrate - (TINOGUARD Q) | | | |
| Eau | qsp 100 % | qsp 100 % | qsp 100 % |

| | | | |
|---|---|---|---|
| (*) hors invention | | | |

### Résultats :

### Essai 1a :

Résultat à To : obtention d'un parfum de couleur jaune d'or et odeur agréable.
Résultat de suntest 16 h : dégradation de la couleur = décoloration --
Observation 2 mois 45°C : altération très nette de l'odeur, odeur piquante, --

### Essai 1 b :

Résultat à To : obtention d'un parfum de couleur jaune d'or et odeur agréable
Résultat de suntest 16 h : dégradation de la couleur = décoloration ---
Observation 2 mois 45°C : dégradation de l'odeur ---

### Essai 1c (selon l'invention):

Résultat à To : obtention d'un parfum de couleur jaune d'or et odeur agréable
Résultat de suntest 16 h : Maintien de la couleur initiale.
Observation 2 mois 45°C : Aucune évolution d'odeur.

On observe que la composition 1c selon l'invention comprenant l'association 2-(4-diéthylamino-2-hydroxybenzoyl)-benzoate de n-hexyle/ Ethylhexyl Methoxycinnamate/ Tris-(tétraméthylhydroxypipéridinol) présente une odeur et une couleur stables à l'exposition de lumière et au stockage à 45°C contrairement aux compositions 1a et 1b (hors invention).

### Exemples 2a, 2b et 2c

| **Composition** | **2a (*)** | **2b (*)** | **2c (invention)** |
|---|---|---|---|
| Concentré de parfum x | 15 % | 15 % | 15 % |
| Ethanol | 65 % | 65 % | 65 % |
| Colorants | qs | qs | qs |
| Mélange de 2-(4-diéthylamino- | 1 | 0 % | 1 |
| 2-hydroxybenzoyl)-benzoate de | | | |
| n-hexyle et de Ethylhexyl | | | |
| Methoxycinnamate 35/65 | | | |
| (Uvinul A + B) | | | |
| Bumetrizole - composé (4) | 0 | 0,03 | 0,03 |
| (TINOGUARD AS) | | | |
| Eau | qsp 100 % | qsp 100 % | qsp 100 % |

| | | | |
|---|---|---|---|
| (*) hors invention | | | |

### Résultats :

### Essai 2a :

Résultat à To : obtention d'un parfum de couleur jaune d'or et odeur agréable.
Résultat de suntest 16 h : dégradation de la couleur = décoloration --
Observation 2 mois 45°C : altération très nette de l'odeur, odeur piquante, --

### Essai 2b :

Résultat à To : obtention d'un parfum de couleur jaune d'or et odeur agréable
Résultat de suntest 16 h : dégradation de la couleur = décoloration ---
Observation 2 mois 45°C : dégradation de l'odeur ---

### Essai 2c (selon l'invention):

Résultat à To : obtention d'un parfum de couleur jaune d'or et odeur agréable
Résultat de suntest 16 h : Maintien de la couleur initiale.
Observation 2 mois 45°C : Aucune évolution d'odeur.

On observe que la composition 2c selon l'invention comprenant l'association 2-(4-diéthylamino-2-hydroxybenzoyl)-benzoate de n-hexyle/ Ethylhexyl Methoxycinnamate/ Bumetrizole présente une odeur et une couleur stables à l'exposition de lumière et au stockage à 45°C contrairement aux compositions 2a et 2b (hors invention).

### Exemples 3a, 3b, 3c et 3d

| **Composition** | **3a(*)** | **3b(*)** | **3c(*)** | **1d (invention)** |
|---|---|---|---|---|
| Concentré de parfum x | 15 % | 15 % | 15 % | 15 % |
| Ethanol | 65% | 65 % | 65% | 65% |
| Colorants | qs | qs | qs | qs |
| 2-(4-diéthylamino-2- | 1 % | 0 | 0 | 0 |
| hydroxybenzoyl)-benzoate de | | | | |
| n-hexyle (Uvinul A+) | | | | |
| Mélange de 2-(4-diéthylamino- | 0 | 1 % | 0 | 1 |
| 2-hydroxybenzoyl)-benzoate de | | | | |
| n-hexyle et de Ethylhexyl | | | | |
| Methoxycinnamate 35/65 | | | | |
| (Uvinul A + B) | | | | |
| Butylmethoxydibenzoylmethane | 0 | 0 | 0,3 % | 0,3 |
| (Parsol 1789) | | | | |
| Eau | qsp 100 % | qsp 100 % | qsp 100 % | qsp 100 % |

| | | | | |
|---|---|---|---|---|
| (*) hors invention | | | | |

### Résultats :

### Essai 3a :

Résultat à To : obtention d'un parfum de couleur jaune d'or et odeur agréable.
Résultat de suntest 16 h : dégradation de la couleur = décoloration ---
Observation 2 mois 45°C: altération très nette de l'odeur, odeur piquante, ---

### Essai 3b :

Résultat à To : obtention d'un parfum de couleur jaune d'or et odeur agréable
Résultat de suntest 16 h : dégradation de la couleur = décoloration --
Observation 2 mois 45°C: dégradation de l'odeur --

### Essai 3 c :

Résultat à To : obtention d'un parfum de couleur jaune d'or et odeur agréable
Résultat de suntest 16 h : dégradation de la couleur = décoloration (---)
Observation 2 mois 45°C : dégradation très nette de l'odeur (---)

### Essai 3 d (selon l'invention):

Résultat à To : obtention d'un parfum de couleur jaune d'or et odeur agréable
Résultat de suntest 16 h : Maintien de la couleur initiale.
Observation 2 mois 45°C : Très faible évolution d'odeur.(-)

On observe que la composition 3d selon l'invention comprenant l'association 2-(4-diéthylamino-2-hydroxybenzoyl)-benzoate de n-hexyle/ Ethylhexyl Methoxycinnamate/ Butylmethoxydibenzoylmethane présente une odeur et une couleur stables à l'exposition de lumière et au stockage à 45°C contrairement aux compositions 3a, 3b et 3c (hors invention).

### Exemples 4a, 4b et 4c

| **Composition** | **4a (*)** | **4b (*)** | **4c (invention)** |
|---|---|---|---|
| Concentré de parfum x | 15 % | 15 % | 15 % |
| Ethanol | 65 % | 65 % | 65 % |
| Colorants | qs | qs | qs |
| Mélange de 2-(4-diéthylamino- | 0 | 1 % | 0,6 |
| 2-hydroxybenzoyl)-benzoate de | | | |
| n-hexyle et de Ethylhexyl | | | |
| Methoxycinnamate 35/65 | | | |
| (Uvinul A + B) | | | |
| Ethylhexyl salicylate (NEO | 0,3% | 0% | 0,1 |
| HELIOPAN OS) | | | |
| Butylmethoxydibenzoylmethane | 0% | 0% | 0,2 |
| (Parsol 1789) | | | |
| Eau | qsp 100 % | qsp 100 % | qsp 100 % |

| | | | |
|---|---|---|---|
| (*) hors invention | | | |

### Résultats :

### Essai 4a :

Résultat à To : obtention d'un parfum de couleur jaune d'or et odeur agréable.
Résultat de suntest 16 h : dégradation de la couleur = décoloration ---
Observation 2 mois 45°C : altération très nette de l'odeur, odeur piquante, ---

### Essai 4b :

Résultat à To : obtention d'un parfum de couleur jaune d'or et odeur agréable
Résultat de suntest 16 h : dégradation de la couleur = décoloration --
Observation 2 mois 45°C : dégradation de l'odeur --

### Essai 4 c (selon l'invention):

Résultat à To : obtention d'un parfum de couleur jaune d'or et odeur agréable
Résultat de suntest 16 h : Maintien de la couleur initiale.
Observation 2 mois 45°C : Aucune évolution d'odeur

On observe que la composition 4c selon l'invention comprenant l'association 2-(4-diéthylamino-2-hydroxybenzoyl)-benzoate de n-hexyle/ Ethylhexyl Methoxycinnamate/ Butylmethoxydibenzoylmethane/ Ethylhexyl salicylate présente une odeur et une couleur stables à l'exposition de lumière et au stockage à 45°C contrairement aux compositions 4a et 4b (hors invention).

## Revendications

1. Composition parfumante comprenant dans un milieu cosmétiquement acceptable :
a) au moins 2% en poids d'une substance parfumante par rapport au poids total de la composition ;
b) au moins un filtre A hydroxyaminobenzophenone de formule (I) 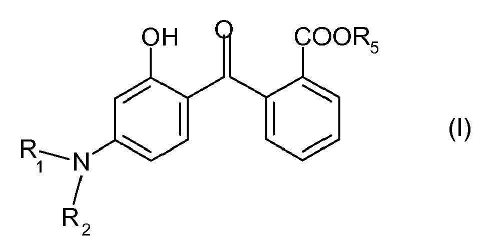 dans laquelle :
R₁ et R₂, identiques ou différents, désignent un atome d'hydrogène, un radical alkyle en C₁-C₂₀ ; un cycloalkyle en C₃-C₁₀ ou un cycloalcényle en C₃-C₁₀ ou forment avec l'atome d'azote avec lequel ils sont liés un cycle à 5 ou 6 chaînons ;
R₃ désigne un radical alkyle en C₁-C₂₀ ;
c) au moins un filtre B cinnamate ;
d) au moins un composé C choisi parmi
(i) un composé pipéridinol de formule (II) suivante : 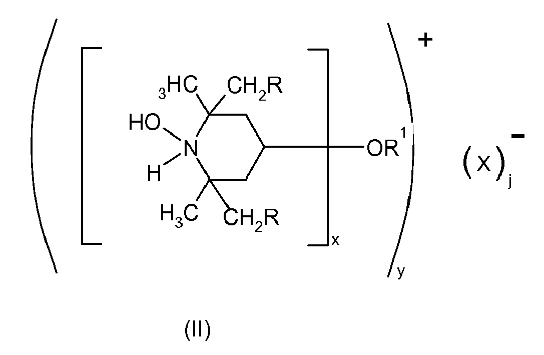 dans laquelle
R désigne hydrogène ou méthyle
x vaut 1 ou 2
1) lorsque x est égal à 1 :
R¹ désigne un hydrogène ; un radical alkyle en C₁-C₁₈ un radical alcényle en C₂-C₁₈ un radical propargyle ; un groupe glycidyle ; un radical alkyle en C₂-C₅₀ interrompu par 1 à 20 atomes d'oxygène, ledit alkyle étant substitué par 1 à 10 groupes hydroxyles ou bien à la fois interrompu par lesdits atomes d'oxygène et substitué par lesdits groupes hydroxyles ; un radical alkyle en C₁-C₄ substitué par un groupe carboxy ou un groupe -COOZ où Z est hydrogène, un alkyle en C₁-C₄, phényle, un alkyle en C₁-C₄ substitué par un groupe (COO ⁻) p M ^{p+} où p est un entier de 1 à 3 et M un ion métallique des groupes 1, 2 et 3 du tableau périodique ou Zn , Cu, Ni ou Co ou bien M est un groupe N^{p+} (R²)₄ où R² est un alkyle en C₁-C₈ ou un benzyle ;
2) lorsque x vaut 2 ,
R' est un radical alkylène en C₁-C₁₂ un radical alcénylène en C₄-C₁₂ ; un groupe xylylène ; un radical alkylène en C₁-C₅₀ interrompu par 1 à 20 atomes d'oxygène ledit alkyle étant substitué par 1 à 10 groupes hydroxyles ou bien à la fois interrompu par lesdits atomes d'oxygène et substitué par lesdits groupes hydroxyles ; X désigne un anion organique ou minéral ;
la charge totale en cations y étant égal à la charge totale en anions j ;
(ii) au moins un composé benzotriazole de formule (III) 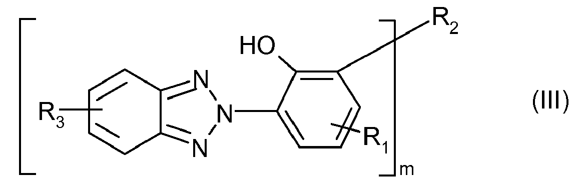 dans laquelle
R₁ désigne un radical alkyle en C₁-C₁₂, un radical alcoxy en C₁-C₅, un alcoxycarbonyle en C₁-C₅, un cycloalkyle en C₅-C₇, un aryle en C₆-C₁₀, un radical aralkyle, un groupe SO₃M, un radical de formule (IIa) 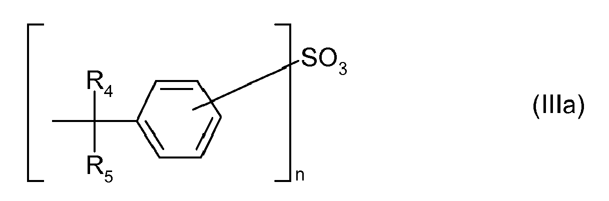 R₄ et R₅, identiques ou différents désignent un alkyle en C₁-C₅ ou un hydrogène ;
R₃ désigne hydrogène, un radical alkyle en C₁-C₁₂, un radical alcoxy en C₁-C₅, un halogène de préférence Cl ou un groupe hydroxy ;
M désigne un cation métallique alcalin, alcalino-terreux ou ammonium de préférence Na+ m désigne 1 ou 2
n vaut 0 ou 1
si m = 1 alors R₂ est hydrogène, un groupe alkyle en C₁-C₁₂ substitué ou non par un phényle ; un aryle en C₆-C₁₀ ;
si m = 2 alors R₂ est une liaison directe, un radical -(CH₂) p- avec p étant un entier de 1 à 3;
(iii) un filtre UV du type dibenzoylméthane.

2. Composition selon la revendication 1, où la quantité de substance parfumante est de 3 à 50 % en poids, mieux de 5 à 30%, encore mieux 10 à 20% en poids par rapport au poids total de la composition.

3. Composition selon l'une quelconque des revendications 1 à 2, où le composé C de formule (I) est le 2-(4-diéthylamino-2-hydroxybenzoyl)-benzoate de n-hexyle de formule (A) suivante :

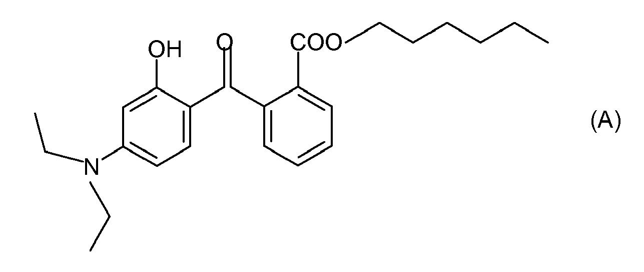

4. Composition selon l'une quelconque des revendications 1 à 3, où le composé B cinnamate est l'Ethylhexyl Methoxycinnamate de formule (B) suivante :

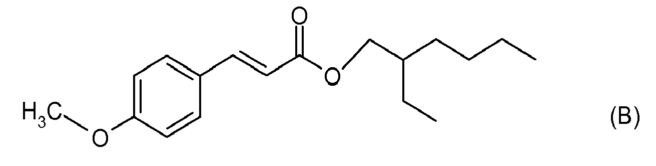

5. Composition selon l'une quelconque des revendications 1 à 4, où la composition contient l'association 2-(4-diéthylamino-2-hydroxybenzoyl)-benzoate de n-hexyle / Ethylhexyl Methoxycinnamate dans un rapport en poids 35/65% en poids.

6. Composition selon l'une quelconque des revendications 1 à 5, où le composé C pipéridinol de formule (II) est le Tris-(tétraméthylhydroxypipéridinol) citrate de structure : 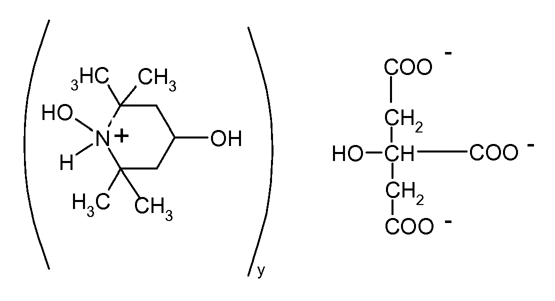 avec y = 3.

7. Composition selon l'une quelconque des revendications 1 à 6, où le composé de C benzotriazole de formule (III) est choisis parmi les composés suivants :
(i) Sodium Benzotriazolyl Btylphenol Sulfonate de formule (1) 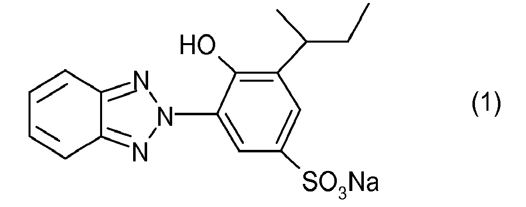
(ii) Benzotriazolyl dodécyl p-Cresol de formule (2) : 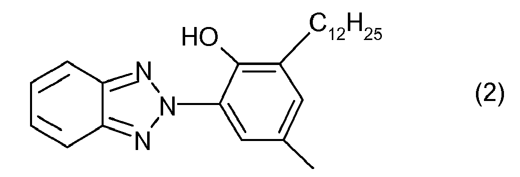
(iii) Benzotriazolyl Butylphenol Sulfonate de formule (3) 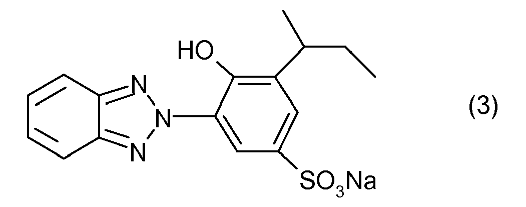
(iv) Bumetrizole de formule (4) :

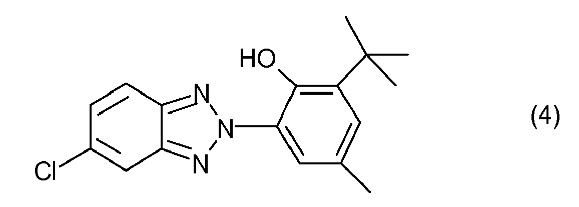

8. Composition selon la revendication 7, où le composé C de formule (II) est le Bumetrizole de formule (4) :

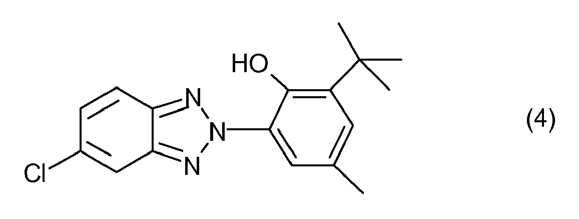

9. Composition selon l'une quelconque des revendications 1 à 8, **caractérisée par le fait que** le composé C est un dibenzoylméthane et qu'elle contient en plus au moins un filtre D salicylate.

10. Composition selon l'une quelconque des revendications 1 à 9, où le compose C dibenzoylméthane est le 4-(ter.-butyl) 4'-méthoxy dibenzoylméthane ou Butyl Methoxy Dibenzoylmethane de formule (IV) suivante :

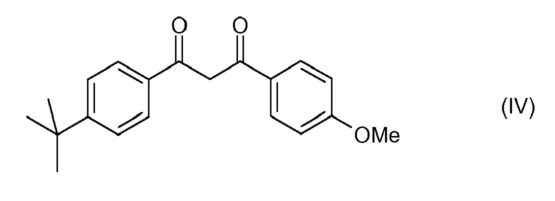

11. Composition selon la revendication 10, où le filtre D salicylate est l'Ethylhexyl Salicylate de formule (V) suivante :

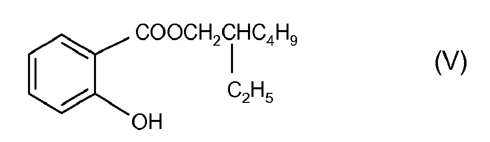

12. Composition selon l'une quelconque des revendications 1 à 11, où le milieu cosmétiquement acceptable contient au moins un alcool volatil et/ou une huile de silicone volatile et éventuellement de l'eau.

13. Composition selon la revendication 12, où le milieu cosmétiquement acceptable contient de l'eau dans une quantité allant de préférence de 0,01 % à 50 % et plus préférentiellement, de 0,5 % à 25 % en poids par rapport au poids total de la composition.

14. Utilisation de l'association d'au moins un filtre A du type hydroxyaminobenzophenone de formule (I), d'au moins un filtre B du type cinnamate et d'au moins un composé C telle que définie dans les revendications précédentes dans une composition parfumante comprenant dans un milieu cosmétiquement acceptable au moins 2% en poids d'une substance parfumante par rapport au poids total de la composition ; comme agent stabilisant des propriétés organoleptiques de la composition vis-à-vis des agressions extérieures comme la lumière ou les différences de température en particulier de la couleur et/ou de l'odeur de ladite composition

15. Procédé cosmétique de parfumage des matières kératiniques humaines et, comprenant l'application sur les matières kératiniques de la composition telle que définie dans les revendications 1 à 13.
